# EUROPEAN PATENT APPLICATION

(11) **EP 3 741 776 A1**
(43) Date of publication of application: **25.11.2020**
(21) Application number: 20179470.8
(22) Date of filing: 20.01.2017
(51) Int. Cl.: C07K 16/02, C07K 16/08, C07K 14/435, A61P 33/00, A61P 33/14, A61K 39/00

(54) **IGY ANTIBODIES FOR THE PREVENTION OF SEA LICE INFESTATION AND INFECTION**

(30) Priority: 22.01.2016 NO 20160113
(62) Divisional of application: 17724461.3
(71) Applicant: Norifish AS, 0105 Oslo (NO)
(72) Inventor: Morlandstø, Jan Ove, 5427 Urangsvåg (NO); Evensen, Øystein, 0198 Oslo (NO); Skjervold, Per Olav, 1430 Ås (NO)
(74) Representative: Acapo AS

(57) **Abstract**

An IgY composition for use in reduction of infestation and/or prevention and/or treatment of infection of a Salmonidae by a parasite of the order Caligidae is described.

## Description

### Field of the invention

The present invention relates to egg yolk or an IgY antibody, and the use of such an antibody for the reduction of infestation and/or prevention and/or treatment of infection of a Salmonidae by a parasite of the family *Caligidae.*

### Background of the invention

*Lepeophtheirus salmonis* or salmon louse belongs to the family *Caligidae* and is a parasitic copepod in salmonids, particularly Atlantic salmon (*Salmo salar* L.). It is found in the northern hemisphere. *L. salmonis* infection has been a significant problem for the aquaculture industry since the 70's and causes significant economic losses and is also an animal welfare problem. The high prevalence of lice in today cages also serves as an infection pressure on wild salmon in the local environment, which is a strong motivation for the aquaculture industry to control and minimize the infection load. *L. salmonis* has been found in farmed fish in Norway, Faroe Islands, Canada, Scotland, Ireland and Japan.

Pathogenicity of the parasite is related to direct physical damage when the infection pressure is high, and there is indication the parasite can serve as vector for various infectious agents like salmon pancreas disease (PD) virus, infectious salmon anaemia virus and *Vibrio salmonicida.* Identification of the pathogen in the louse or associated with the louse is not the same as being a passive or true vector. In addition, the fish resistance to viral infections is possibly reduced as a result of louse immunomodulation and infection may impact fish growth. The clinical picture depends on the host, the number of lice on the fish as well as the developmental stage of the louse.

Host susceptibility is influenced by factors such as stress, nutritional status, immune status and genetic resistance. The mortality in Atlantic salmon is associated with high infection load (number of parasite) and thus release of components from the parasite itself, combined with stress response in salmon at high infestation (release of endogenous cortisol with subsequent immunosuppression) and inadequate immune response against lice. Secondary infections likely play a role, i.e. skin abrasions caused by the parasite facilitate secondary infections.

Vaccination attempts are several although none have been successful so far. In light of the increasing problems with resistance in sea lice, there is an urgent need for alternative control methods and immune intervention is an attractive one and far more sustainable than the current practices of chemical treatments in open cages with release of active ingredient to the surrounding water and the marine environment.

Parasites have complex life cycles and lice are no exception. Sea lice have a direct life cycle, meaning it requires one host to complete its development. The lice must go through eight stages before reaching sexual maturity and development are temperature dependent, approximately 400 degree days, and require high salinity for optimal. After hatching from eggs, lice will undergo two naupilus stages. This is a free-swimming planktonic stage lasts 5-15 days depending on temperature. Then follows the copepodite stage and this is when the lice find a suitable host and attaches. From this stage and until maturation, immune intervention is possible, but different strategies would have to be employed dependent on development stage of the lice. The copepodites will attach to the skin of the fish using special frontal filaments. Predilection sites are on the fins during the early attachment stages, moving over to the skin behind the dorsal fin. There are two chalimus stages and at this stage it is still attached to the host and feed on the skin mucus, cells and excretory products from/in the skin. These stages will take up mucus, skin and underlying tissue through its mouth part and mechanical interaction with the skin. The two pre-adult stages are motile and they can move from one fish to the other. The last stages are preadult and adult and now the sea lice take up also blood/serum from the host to reach maturity, particularly the female to allow the egg strings to mature.

The approach so far (in the literature) in developing a vaccine against sea lice is in several ways different from those sought for bacterial and viral infections. The vaccine aims to prevent replication of the agent in the host for the two latter categories of pathogens, while for sea lice vaccines the aim is to reduce the number of attached lice to the host and also limit the fecundity/production of off-spring. This can be achieved by inhibiting the lice to reproduce or inhibit reproduction ability lice. A 50% reduction in number of sea lice attached could result in an even more pronounced reduction of the infection pressure to the environment.

There have been several attempts to develop a vaccine against sea lice, but today there are no available vaccines with sufficient protective effects (Carpio et al., 2013, Carpio, Y., Garcia, C., Pons, T., Haussmann, D., Rodriguez-Ramos, T., Basabe, L., Acosta, J., Estrada, M.P. 2013. Akirins in sea lice: first steps towards a deeper understanding. Exp Parasitol 135, 188-199).

Atlantic salmon has been immunized with native antigens from an extract made from whole *L. salmonis* parasites. Specific antibody production was recorded for several antigens, but antibodies were not protective when the salmon were then subjected to pre-adult and adult *L. salmonis* infection (Raynard et al., 2002, Raynard, R.S., Bricknell, I.R., Billingsley, P.F., Nisbet, A.J., Vigneau, A., Sommerville, C. 2002. Development of vaccines against sea lice. Pest Manag Sci 58, 569-575).

It is thus an object of the invention to prepare and provide an antibody that is effective in the sea lice reproduction and viability in order to reduce the number of sea lice attachment to salmonids after a challenge by sea lice.

The object is accomplished by providing an IgY composition that is prepared by immunizing egg-laying hens with an antigen not specific to or obtained from a Caligidae sea lice, and the obtained IgY effectively reduces the number of sea lice attached to salmon after challenge.

### Summary of the invention

A first aspect of the present invention relates to an Egg yolk from an egg from an egg-laying poultry for use in prevention of infestation and/or infections of a Salmonidae, wherein the egg-laying poultry is not immunized.

Preferably, the egg yolk is administered to a Salmonidae and reduces the infestation and/or infection of a Caligidae.

Preferably, the egg yolk comprises an IgY antibody.

A second aspect of the present invention relates to an IgY antibody composition for use in prevention and/or treatment of infestation and/or infections of a Salmonidae by a Caligidae sea lice, wherein said IgY composition is obtained from the egg yolk from an egg-laying poultry, wherein the egg-laying poultry is not immunized, or is not immunized with a virus or a Caligidae sea lice, or portions thereof.

A third aspect of the present invention relates to a pharmaceutical composition comprising an IgY antibody composition as indicated above, and at least one medically compatible adjuvant.

### Description of the drawings

Fig. 1. Sea lice numbers at 27 and 45 days post infection in different treatment groups. Statistical analysis was performed for IgY versus control fish and the results are shown below. Two-sided t-test gives p<0.0000.
Fig. 2. Sea lice numbers at 8, 15 and 32 days post infection (dpi) in different treatment groups. Grey=IgY group; maroon=IgY-NS, and green=control fish.
Fig. 3. Neutralizing ability of IgY antibody against Nodavirus was expressed by virus fold reduction relative to control (virus without antibody) measured by qPCR
Fig. 4. The neutralizing effect of IgY where a 1:1000 dilution of IgY (20 microgram) will neutralize 1000 virus particles.

### Description of embodiments of the invention

### Experimental section

### Example 1

### Antigen preparation and immunization protocols

Salmon louse, *Lepeophtheirus salmonis,* of different development stages, copepodids/chalimus, pre-adult and adult sea lice with egg-strings, were collected from infected salmon on the west coast of Norway (Hordaland county). The different stages of lice where collected from anesthetized salmon, and transferred to phosphate-buffered formalin (10% w/w) and stored in formalin at 4C until processed.

Antigen preparations were made in two different batches, including batch 1), copepodids and chalimus stages, and batch 2) pre-adult and adult stage (male and female; equal proportions). Approximately 5 lice of each stage were pooled into batch 1) and 2) and homogenized in sterile phosphate buffered saline (PBS), pH=7.2. The batches were pooled, and the homogenates were centrifuged at 2500 x *g* for 5 min (at 4C) and the supernatant was collected (aspirated) using sterile pipettes. The protein concentration was measured using standard methods (Bradford) and adjusted to 20 mg/ml for batch 1 and 40 mg/ml for batch 2.

Chickens (n=6) at egg-laying age (20 weeks old) were injected intramuscularly 4 times, 21 days apart by the antigen preparations.. The first injection was made with the antigen preparation (as described above), mixed 1:1 (weight:weight) with Freund's complete adjuvant and given at a dose of 0.5 ml per chicken (intramuscularly, *Musculus pectoralis majoris*)*.* Subsequent injections were made using Freund's incomplete adjuvant mixed as described. Injection volume was 0.5 ml per chicken. When the hens started egg-laying, extraction of IgY was performed as follows. Twice the egg yolk volume of PBS was mixed with yolk, thereafter 3.5 % polyethylene glycol (PEG) 6000 (in gram) of the total volume was added and vortexed, followed by 10 min rolling on a rolling mixer. The watery phase was collected, ant this watery phase contained the IgY and other proteins. This is followed by centrifugation (3000 rpm) at 4°C, 20 min. The supernatant is poured through a folded filter (0.4 micron) and transferred to a new tube. 8.5 % (w/v) PEG 6000 is added to the tube, the tube is vortexed and rolled on a rolling mixer (max 30 s). This step is repeated and the supernatant is discarded. The pellet is carefully dissolved in 1 ml PBS using the vortexer. PBS is added to a final volume of 10 ml. The solution is mixed with 12 % PEG 6000 (weight/vol, 1.2 gram) and then vortexed.

The pellet was then dissolved carefully in 800 µl PBS (glass stick and vortex) and transferred to a dialysis capsule. The extract was dialysed (holding back >50 kDa size) over night in 0.1 % saline (1,600 ml) and gently stirred using a magnetic stirrer. The next morning, the saline is replaced by PBS and dialysed for another three hours. The IgY-extract was pulled from the dialysis capsule and transferred to 50 ml tubes.

The protein content (mg/mL) of the samples was measured photometrically at 280 nm (1:50 diluted with PBS) and calculated according to the Lambert-Beer law with an extinction coefficient of 1.33 for IgY.

### Immunization of salmon and challenge

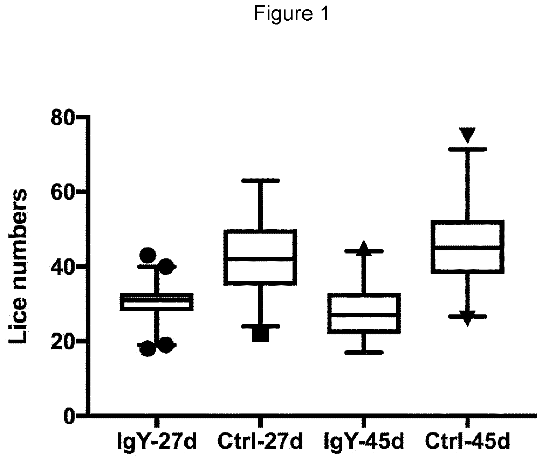

Atlantic salmon (*Salmo salar* L.), AquaGen AS source (obtained from Sør-Smolt, Norway), of 300-350g size, kept in sea water (8 C). The fish were kept in two different tanks (n=34 in each; Fig. 1). The fish were injected with 0.05-0.06 mg IgY/g live weight (1:1 v/v of batch 1 and 2 above), intraperitoneally, using a 24G needle, 1 day prior to challenge with sea lice copepodids. Non-treated controls (n=34 fish per tank) were run in 2 parallel tanks.

90 nauplii of *Lepeophtheirus salmonis,* per fish were added to a tank containing 200L of seawater (32‰). The water flow was turned off during the challenge with the sea lice, and the water was oxygenated through an external source and monitoring continuously using a standard oxygen probe. The concentration of oxygen was not allowed to fall below 8%. The copepodids were allowed to attach to the fish for 30 minutes after which the water flow was resumed and oxygenation ceased.

### Counting of sea lice

Counting of sea lice of different stages was done at 21 days post challenge and then at 42 days post challenge.

At 25 days post challenge, the non-treated controls fish had an average of 45 sea lice (total) per fish while the IgY-treated fish had an average of 29 sea lice/fish; giving a reduction of 35.6% (Fig. 1). At 45 days post challenge, the number of sea lice in the controls was 45/fish while the IgY-treated fish had 28 sea lice/fish, i.e. a 38% reduction (Fig. 1). The experiment was completed at 45 days post challenge and the fish were deloused.

Table 1 shows the statistical comparison between IgY and controls at 27 days post infection. Data were tested for normality by Shapiro-Wilks test (Stata14). Same results were obtained for the 45 days post infection data.

**Table 1**

| Two-sample t test with unequal variances | | | | | | |
|---|---|---|---|---|---|---|
| Variable | Obs | Mean | Std. Err. | Std. Dev. | [95% Conf. Interval] | |
| Ctrl27d | **40** | **42.9** | **1.626227** | **10.28516** | **39.61064** | **46.18936** |
| IgY27d | **40** | **30** | **.8764552** | **5.505242** | **28.23934** | **31.76066** |
| combined | **80** | **36.45** | **1.168941** | **10.45533** | **34.12328** | **38.77672** |
| diff | | **12.9** | **1.844535** | | **9.21127** | **16.58873** |
| diff = mean(Ctrl27d) - mean(IgY27d) | | | | t = **6.9936** | | |
| Ho: diff = 0 | | | Welch's degrees of freedom = **60.7112** | | | |
| Ha: diff < 0 | | Ha: diff ! = 0 | | | Ha: diff > 0 | |
| Pr(T < t) = **1.0000** | | Pr(\|T\| > \|t\|) = **0.0000** | | | Pr(T > t) = **0.0000** | |

This experiment clearly shows that the administration of IgY antibodies produces after a challenge with L. salmonis inhibit that infestation and/or attachment of sea lice to the sea lice challenged fish, and can thus be used as a prophylactic and/or curative measure against sea lice infection. This invention is the subject of a co-pending patent application.

### Example 2

### Antigen preparation and immunization protocols

The sea lice antigen preparation was made as described above and the same immunization protocol was used as described in example 1. This IgY preparation is referred to as IgY. In addition, we have prepared an IgY preparation obtained from immunization and purification protocols as described above, where the antigen was salmon pancreas disease virus (obtained and prepared as described in (Xu *et al.,* 2012). This preparation is referred to as non-specific IgY, (Ig-NS). The antibody produced by this non-specific immunization is the subject invention of the present application.

### Immunization of salmon and challenge

Atlantic salmon, 85 g, was obtained from Sør-Smolt (AquaGen AS source). The fish were vaccinated against furunculosis, vibriosis, cold-water vibriosis and winter ulcer. The fish were transported to the experimental facility, and allowed to be acclimatized for 4 weeks prior to transfer to the experimental challenge unit. Fish were transferred to 2 separate tanks, 39 fish per tank. Water level was kept at 400L per tank, 32‰ seawater, 8°C. In tank 1, 19 fish were injected with IgY and 20 fish with IgY-NS. The IgY-NS fish were marked by fin-clipping. In tank 2, 20 fish were injected with IgY and 19 fish with IgY-NS, and this group was fin-clipped. Control fish (non-treated) were kept in 3 parallel tanks, containing 45, 43 and 39 fish per tank.

Counting of sea lice was done as described in Experiment 1, and at 8, 15, 21, and 32 days post challenge. The results are shown in Table 2.

**Table 2**

| Groups | Days post challenge (# of lice counted) | | | |
|---|---|---|---|---|
| | 8 | 15 | 21 | 32 |
| IgY | 4.5 (n=3) | 4.7 (n=6) | 7.1 (n=6) | 4.2 (n=37) |
| IgY-NS | 5.5 (n=3) | 8.2 (n=6) | 10.5 (n=6) | 8.6 (n=38) |
| Control | 7.5 (n=6) | 14.9 (n=18) | 16.9 (n=18) | 13.9 (n=127) |
| | | | | |
| **% reduction IgY** | **40** | **68.5** | **58** | **69.8** |
| % reduction IgY-NS | 26.7 | 45 | 37.9 | 38.1 |

| | | | | |
|---|---|---|---|---|
| Table 2. The numbers are kept lower at early time points since the fish have to be anesthetized and taken out of the water for 2-3 min for counting. This will impact on the number of lice attached and can result in lice falling off. n = number of fish counted at each time point. | | | | |

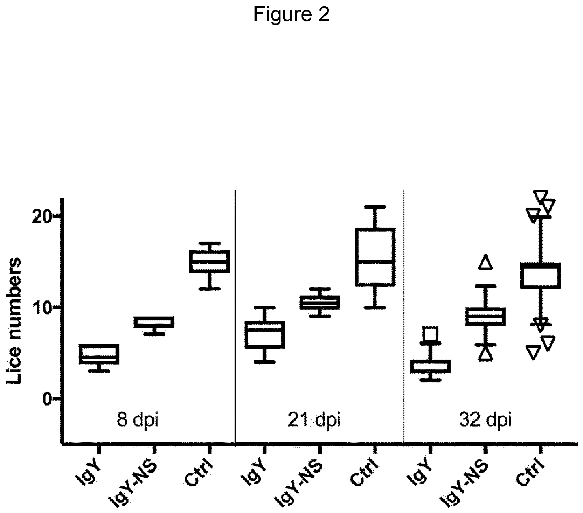

Figure 2 shows the sea lice numbers at 8, 15 and 32 days post infection (dpi) in different treatment groups. Grey=IgY group; maroon=IgY-NS, and green=control fish.

Table 3 shows the statistical analysis performed for IgY versus control fish (on log-transformed data). Two-sided t-test gives p<0.0000. The same was found for IgY versus IgY-NS groups (not shown). Significant difference was found at all time points.

**Table 3**

| Two-sample t test with equal variances | | | | | | |
|---|---|---|---|---|---|---|
| Variable | Obs | Mean | Std. Err. | Std. Dev. | [95% Conf. Interval] | |
| logctrl | **60** | **2.60792** | **.0330102** | **.2556956** | **2.541867** | **2.673973** |
| logIgY | **38** | **1.177097** | **.0598753** | **.3690959** | **1.055778** | **1.298416** |
| combined | **98** | **2.053111** | **.077114** | **.7633893** | **1.900061** | **2.206161** |
| diff | | **1.430823** | **.0631185** | | **1.305534** | **1.556112** |
| diff = mean**(logctrl)** - mean **(logIgY)** | | | | t = **22.6689** | | |
| Ho: diff = 0 | | | | degrees of freedom = **96** | | |
| | | | | | | |
| Ha: diff < 0 | | Ha: diff ! = 0 | | | Ha: diff > 0 | |
| Pr(T < t) = **1.0000** | | Pr(\|T\| > \|t\|) = **0.0000** | | | Pr(T > t) = **0.0000** | |

In summary, the results show that pre-treatment with anti-lice IgY (0.05 mg/g live weight of fish) gives a highly significant reduction in number of sea lice at all stages from 8 till 32 days post infection. The reduction in sea lice attached to the fish is almost 70% 15 and 32 days after challenging of the fish with sea lice, and the effect is patented in a co-pending application.

The results also show that the IgY-NS antibody (i.e. an IgY antibody with an irrelevant specificity) reduces the number of sea lice attached to the fish. The reduction obtained by administration of the IgY-NS, with regard to sea lice attached to the fish is 38-40% after 15, 21 and 32 days after challenging of the fish with sea lice.

### Example 3

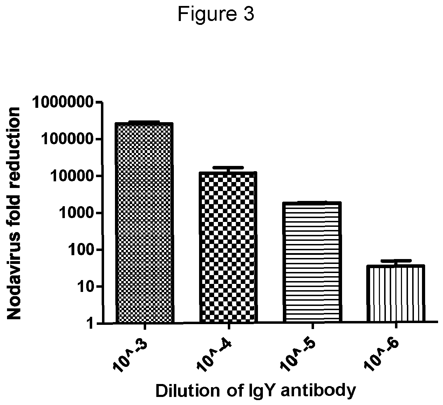

In this study IgY antibodies against nodavirus was produced as described in Example 1. The concentration of virus used for immunization was 10¹⁰ virus particles per ml of solution. IgY was harvested as described in Example 1 and 500 µl of serial diluted anti-nodavirus IgY antibody was mixed with 500 µl of 100 TCID₅₀ per ml of nodavirus. The mixtures were incubated for 1 h at room temperature and subsequently added to confluent AGK cells in 24-well plates. At day 4 post-infection, culture supernatants were collected and RNA extracted. Expression of viral coat protein gene transcription was measured by qPCR. Neutralizing ability of IgY antibody against Nodavirus was expressed by virus fold reduction relative to control (virus without antibody) measured by qPCR, as shown in figure 3.

The IgY antibodies can be diluted more than 10 000 times result in a 10 000 times reduction in virus replication levels (measured by real time PCR). At this dilution of IgY there was a minor cytopathic effect in the cell cultures as an indication of almost complete neutralization of the infection.

### Example 4

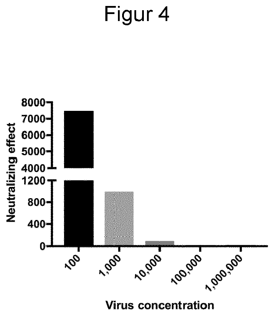

IgY antibodies were produced in chicken as described in Example 1 and Example 3, using 10¹⁰ virus particles per ml of vaccine preparation for chicken. Egg yolk was collected as described and IgY solution prepared as described in Example 1, at a final concentration of 20 mg/ml. IgY solution was diluted 1:10 (in phosphate buffered saline) up to 1:10 000 000 (10⁻⁷) and mixed with increasing concentration of virus from 100 and up to 1 000 000 virus particles. The neutralizing effect of IgY is as given in the figure 4 where a 1:1000 dilution of IgY (20 microgram) will neutralize 1000 virus particles.

### Numbered embodiments described in this application

1. IgY antibody composition for use in prevention and/or treatment of infestation and/or infections of a Salmonidae by a Caligidae sea lice, wherein said IgY composition is obtained by immunization of an egg-laying poultry with a virus or a portion of a virus, and wherein said IgY composition is administered to said Salmonidae.
2. IgY antibody composition according to embodiment 1, wherein said virus is a virus that infects marine organisms.
3. IgY antibody composition according to embodiment 2, wherein said virus is salmon pancreas disease (PD) virus.
4. IgY antibody composition according to embodiment 3, wherein said PD virus is an Alphavirus of the species salmon pancreas disease virus, isolated from an Atlantic salmon heart specimen.
5. IgY antibody composition according to embodiment 2, wherein said virus is a nodavirus.
6. IgY antibody composition according to embodiment 5, wherein said nodavirus is a betanodavirus named Red-spotted grouper nervous necrosis virus grouper fish.
7. IgY antibody composition according to embodiment 6, wherein the Caligidae is a Lepeophtheirus.
8. IgY antibody composition according to embodiment 7, wherein the Lepeophtheirus is
   *Lepeophtheirus salmonis.*
9. IgY antibody composition according to embodiment 6, wherein the Caligidae is a Caligus.
10. IgY antibody composition according to embodiment 9, wherein the Caligus is Caligus rogercresseyi.
11. IgY antibody composition according to any of the embodiments 1-10, wherein the Salmonidae is selected from the group consisting of salmon, charr and trout.
12. IgY composition according to embodiment 11, wherein said Salmonidae is Atlantic salmon.
13. IgY composition according to embodiment 11, wherein said Salmonidae is Atlantic salmon smolt.
14. IgY composition according to embodiment 11, wherein said Salmonidae are Oncorhynchus species.
15. A pharmaceutical composition comprising an IgY antibody composition according to any of the embodiments 1-14, and at least one medically compatible adjuvant.
16. A vaccine composition comprising an IgY antibody composition according to any of the embodiments 1-14.
17. Egg yolk from an egg from an egg-laying poultry for use in prevention of infestation and/or infections of a Salmonidae, wherein the egg-laying poultry is not immunized.
18. Egg yolk according to embodiment 1, wherein the egg yolk is administered to a Salmonidae and reduces the infestation and/or infection of a Caligidae.
19. Egg yolk according to embodiment 1, wherein the egg yolk comprises an IgY antibody.
20. IgY antibody composition for use in prevention and/or treatment of infestation and/or infections of a Salmonidae by a Caligidae sea lice, wherein said IgY composition is obtained from the egg yolk from an egg-laying poultry, wherein the egg-laying poultry is not immunized, or is not immunized with a virus or a Caligidae sea lice, or portions thereof.

## Claims

1. Egg yolk from an egg from an egg-laying poultry for use in prevention of infestation and/or infections of a Salmonidae, wherein the egg-laying poultry is not immunized.

2. Egg yolk according to claim 1, wherein the egg yolk is administered to a Salmonidae and reduces the infestation and/or infection of a Caligidae.

3. Egg yolk according to claim 1, wherein the egg yolk comprises an IgY antibody.

4. IgY antibody composition for use in prevention and/or treatment of infestation and/or infections of a Salmonidae by a Caligidae sea lice, wherein said IgY composition is obtained from the egg yolk from an egg-laying poultry, wherein the egg-laying poultry is not immunized, or is not immunized with a virus or a Caligidae sea lice, or portions thereof.

5. A pharmaceutical composition comprising an IgY antibody composition according to claim 4, and at least one medically compatible adjuvant.
